# EUROPEAN PATENT SPECIFICATION

(11) **EP 2 895 138 B1**
(45) Date of publication and mention of the grant of the patent: **22.11.2017**
(21) Application number: 13759986.6
(22) Date of filing: 11.09.2013
(51) Int. Cl.: A61K 6/083

(54) **DENTAL COMPOSITION**
DENTALE ZUSAMMENSETZUNG
COMPOSITION DENTAIRE

(30) Priority: 11.09.2012 EP 12006385
(43) Date of publication of application: 22.07.2015
(73) Proprietor: Dentsply DeTrey GmbH, 78467 Konstanz (DE)
(72) Inventor: KLEE, Joachim E., 78315 Radolfzell (DE); GANSEL, Julia, 76646 Bruchsal (DE); FIK, Christoph P., 9215 Schönenberg a.d. Thur (CH); WAGNER, Caroline, 78224 Singen (DE); ELSNER, Oliver, 78315 Radolfzell (DE); MAIER, Maximilian, 78462 Kontanz (DE); LAMPE, Ulrich, 56727 Mayen (DE); RITTER, Helmut, 42111 Wuppertal (DE); KAVALLAR, Lisa-Maria, 46049 Oberhausen (DE)
(74) Representative: Hartz, Nikolai
(86) International application number: PCT/EP2013/002731
(87) International publication number: WO 2014/040729

(56) References cited:
- WO-A2-2008/015646
- US-A1- 2007 129 458

## Description

### Field of the Invention

The present invention relates to a dental composition comprising a specific polymerizable compound. Furthermore, the present invention relates to the use of the specific polymerizable compound for the preparation of a dental composition. The polymerizable compound of the present invention is a water soluble yet hydrolysis stable polyfunctional polymerizable monomer, which is copolymerizable with conventional (meth)acrylates and provides dental compositions with a low viscosity and excellent biocompatibility.

### Background of the Invention

WO2008015646 discloses hair coloring or bleaching compositions and kits thereof comprising at least one cross-linked amphoteric polymer thickener obtained by polymerizing a mixture which may contain N,N-diallylacrylamide as a crosslinking agent. A dental composition containing N,N-diallylacrylamide is not disclosed in WO2008015646.

Dental compositions containing polymerizable compounds are known. Conventionally, polymerizable dental compositions are provided for a broad range of applications and must, therefore, meet diverse requirements. For example, a polymerizable dental composition may be a dental adhesive composition, a bonding agent, a pit and fissure sealant, a dental desensitizing composition, a pulp capping composition, a dental composite, dental glass ionomer cement, a dental cement or a dental root canal sealer composition. Moreover, a dental composition may be a dental infiltrant.

Conventionally, (meth)acrylates are used as polymerizable components in polymerizable dental compositions due to their excellent reactivity in radical polymerizations which may be demonstrated based on the polymerization enthalpy in the range of from Δ_{R}H = - 80 to 120 kJ/mol. In order to provide crosslinking capability, polyfunctional (meth)acrylates such as bis-GMA, were used for dental applications as early as 1962.

However, hydrolysis stability of (meth)acrylates is problematic in view of the acidity of many dental compositions which severely limits the storage stability of the dental composition. Moreover, hydrolysis taking place under biological conditions in the mouth of the patient is a further concern regarding (meth)acrylates.

EP1234553 discloses (meth)acrylamides having an increased hydrolysis stability. However, certain (meth)acrylamides have an increased viscosity as compared to the corresponding (meth)acrylates which is undesireable in the case of dental compositions wherein the polymerizable matrix is required to have a low viscosity.

Allyl derivatives including allyl amine are usually not suitable as polymerizable compounds for dental compositions since the contribution of the allyl group to the heat of polymerization in a radical reaction is too low even in a copolymerization reaction with (meth)acrylates.

### Summary of the Invention

It is the problem of the present invention to provide a dental composition comprising a hydrolysis stable polyfunctional polymerizable monomer, which is copolymerizable with conventional (meth)acrylates and which has a low viscosity and excellent biocompatibility.

The present invention provides a dental composition comprising a polymerizable compound of the following formula (I):

A-L(B)ₙ (I)

wherein
- A: is a group of the following formula (II) 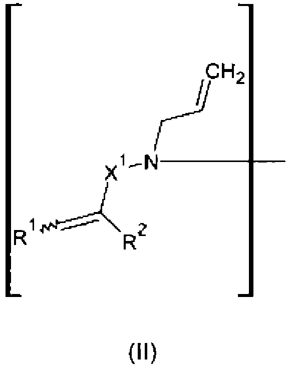
- X¹: is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10;
- R¹: is a hydrogen atom,
-COOM,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M,
a C₆₋₁₄ aryl or C₃₋₁₄ heteroaryl group which may be substituted by -COOM, -PO₃M, - O-PO₃M₂ or -SO₃M,
- R²: is a hydrogen atom,
-COOM
a straight chain or branched C₁₋₁₆ alkyl group which may be substituted by a C₆₋₁₄ aryl or C₃₋₁₄ heteroaryl group, -COOM, -PO₃M, -O-PO₃M₂ and -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, or
a C₆₋₁₄ aryl or C₃₋₁₄ heteroaryl group which may be substituted by -COOM, -PO₃M, - O-PO₃M₂ and -SO₃M,
- L: is a single bond or a linker group;
- B: independently is
a group according to the definition of A,
a group of the following formula (III) 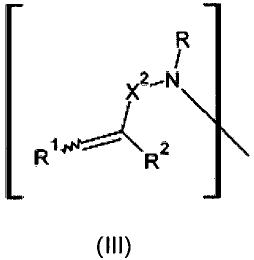 wherein
X² independently has the same meaning as defined for X¹ in formula (II),
R¹ and R² are independent from each other and independently have the same meaning as defined for formula (II),
R is a hydrogen atom, a group a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, - O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M,
a C₆₋₁₄ aryl group which may be substituted by -COOM, -PO₃M, -O-PO₃M₂ or -SO₃M,
a group of the following formula (IV) 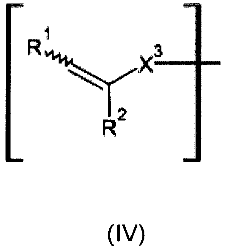 wherein
X³ is CO, -CH₂CO-, CS, or -CH₂CS-,
R¹ and R² which are independent from each other and independently have the same meaning as defined for formula (II), or a group [Z'X"]ₘE,
wherein
Z' is a straight chain or branched C₁₋₄ alkylene group,
X" is an oxygen atom, a sulfur atom or NH,
E is a hydrogen atom,
PO₃M₂,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, - PO₃M, -O-PO₃M₂ or -SO₃M,
a C₆₋₁₄ aryl or C₃₋₁₄ heteroaryl group which may be substituted by -COOM, -PO₃M, -O-PO₃M₂ or -SO₃M, and
m is an integer of from 1 to 10;
and
n is an integer of from from 1 to 4;
wherein M which are independent from each other each represent a hydrogen atom or a metal atom;
provided that in case L is a single bond, B cannot be a group according to the definition of A or a group of the formula (III), and provided that the compound of formula (I) is not N,N-diallylacrylamide.

The present invention also provides a use of a compound of formula (I) for the preparation of a dental composition selected from a dental adhesive composition, a bonding agent, a pit and fissure sealant, a dental desensitizing composition, a pulp capping composition, a dental composite, dental glass ionomer cement, a dental cement and a dental root canal sealer composition. The present invention also provides a use of a compound of formula (I) for the preparation of a dental infiltrant.

The present invention is based on the recognition that a compound of formula (I) has a heat of polymerization which is comparable to the heat of polymerization of (meth)acrylates despite the presence of allyl groups. Moreover, the present invention is based on the recognition that the viscosity of the compounds of formula (I) is comparably low as compared to the corresponding (meth)acrylamides.

### Detailled Description of Preferred Embodiments

In this description, unless otherwise specified, a halogen atom denotes a fluorine atom, a chlorine atom, a bromine atom or a iodine atom; an alkyl group denotes, for example, a straight-chain or branched-chain C₁₋₁₆ alkyl group such as methyl, ethyl, propyl, isopropyl, butyl, sec-butyl, isobutyl, tert-butyl, pentyl, hexyl and octyl; a cycloalkyl group denotes a C₃₋₆ cycloalkyl group such as cyclopropyl, cyclobutyl, cyclopentyl and cyclohexyl; an aryl group denotes a C₆₋₁₄ aryl group such as phenyl, naphthyl; a heteroaryl group denotes a C₃₋₁₄ heteroaryl group which may contain 1 to 3 heteroatoms selected from nitrogen, oxygen and sulfur, such as furyl, thienyl, pyrrolyl, imidazolyl, pyrazolyl, thiazolyl, isothiazolyl, oxazolyl, isoxazolyl, thiadiazolyl, furazanyl, pyrrolidinyl, imidazolidinyl, pyridyl, pyrimidinyl, pyridazinyl, pyrazinyl, piperazinyl, piperidyl, morpholinyl, thiomorpholinyl, benzofuranyl, isobenzofuranyl, benzothienyl, indolinyl, isoindolinyl, indolizinyl, benzimidazolyl, benzoxazolyl, benzisoxazolyl, benzothiazolyl, benzoisothiazolyl, purinyl, coumarinyl, phthalazinyl, naphthyridinyl, quinoxalinyl, quinazolinyl, cinnolinyl, chromanyl, isochromanyl, chromenyl, thiochromenyl, quinuclidinyl, 1,3-benzodioxolyl, benzothiadiazolyl, benzoxadiazolyl, benzodioxanyl, quinolyl, isoquinolyl; an alkylene group denotes a C₁₋₄ alkylene group such as methylene, ethylene, propylene, and butylene.

The present invention provides a dental composition. The dental composition of the present invention is polymerizable or copolymerizable by a radical polymerization. The dental composition may be selected from a dental adhesive composition, a bonding agent, a pit and fissure sealant, a dental desensitizing composition, a pulp capping composition, a dental composite, dental glass ionomer cement, a dental cement and a dental root canal sealer composition. The dental composition may also be a dental infiltrant.

A dental composition according to the present invention comprises a polymerizable compound of the following formula (I). The dental composition may comprise one or more compounds of formula (I). The dental composition of the present invention may comprise the polymerizable compound in an amount of from 0.5 to 99 percent by weight based on the total weight of the composition. Preferably, a dental adhesive composition comprises 2 to 50 percent by weight of one or more compounds of formula (I). Preferably, a bonding agent comprises 2 to 70 percent by weight of one or more compounds of formula (I). Preferably, a pit and fissure sealant comprises 2 to 80 percent by weight of one or more compounds of formula (I). Preferably, a dental desensitizing composition comprises 2 to 90 percent by weight of one or more compounds of formula (I). Preferably, a pulp capping composition comprises 2 to 50 percent by weight of one or more compounds of formula (I). Preferably, a dental composite comprises 2 to 50 percent by weight of one or more compounds of formula (I). Preferably, a dental glass ionomer cement comprises 2 to 50 percent by weight of one or more compounds of formula (I). Preferably, a dental cement comprises 2 to 50 percent by weight of one or more compounds of formula (I). Preferably, a dental infiltrant comprises 1 to 50 percent by weight of one or more compounds of formula (I).

A compound of formula (I) is accordig to the following formula (I):

A-L(B)ₙ (I)

In formula (I), A is a specific polymerizable group which is linked by a single bond to a single group B or by a linking group to up to four groups B. In case more than one group B is present in the polymerizable compound of formula (I), the groups B may be the same or different. The groups B may be polymerizable.

In order to avoid a nitrogen-nitrogen single bond, B cannot be a group according to the definition of A or a group of the following formula (III) in case L is a single bond.

According to the present invention, A is a group of the following formula (II)

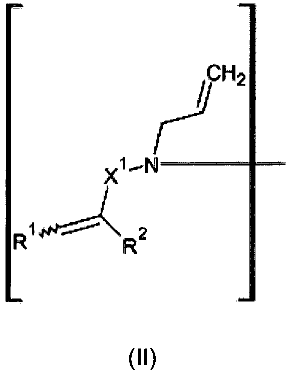

Accordingly, any compound of formula (I) is characterized by an allyl group bonded to a nitrogen atom and a further specific group having a polymerizable double bond which is bonded to the same nitrogen atom. The specific arrangement of the polymerizable double bond of the allyl group and a further polymerizable double bond which is bonded to the same nitrogen atom activates the allyl bond so that the allyl bond takes part in the radical polymerization reaction during curing. Given that an allyl group usually cannot be radically polymerized, it is surprising that the allyl group of a compound of the present invention contributes to the heat of polymerization of the polymerizable compound of formula (I) similar to a methacrylate group.

In formula (II), X¹ is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10.

In case X¹ is CO, CS, CH₂ in formula (II), a polymerizable double bond is present which may take part with the allyl group directly bonded to the nitrogen atom of the group of formula (II) in a cyclopolymerization reaction according to the following scheme.

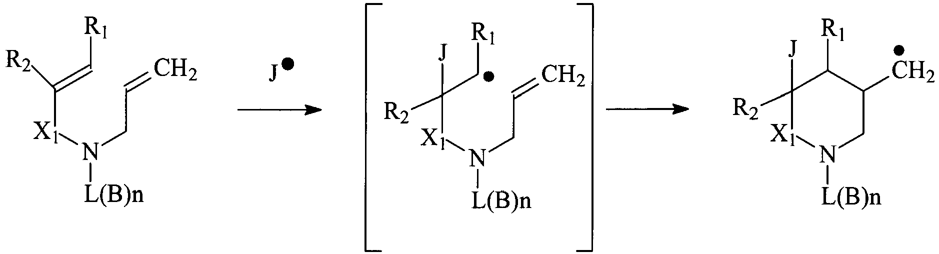

In case X¹ is a group [X'Z]ₖ in formula (II), it is preferred that L(B)ₙ provides a polymerizable double bond is present which may take part with the allyl group directly bonded to the nitrogen atom of the group of formula (II) in a cyclopolymerization reaction. Preferably, X' is an oxygen atom. Preferred examples for a straight chain or branched C₁₋₄ alkylene group for Z are an ethylene group and a propylene group. Preferably, k is an integer of from 1 to 4.

According to a preferred embodiment, X¹ is CO.

In formula (II), R¹ is a hydrogen atom, -COOM, a straight chain or branched C₁₋₁₆ alkyl group which may be substituted by a C₃₋₆ cycloalkyl group, a C₆₋₁₄ aryl or C₃₋₁₄ heteroaryl group,-COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or-SO₃M, a C₆₋₁₄ aryl or C₃₋₁₄ heteroaryl group which may be substituted by -COOM, -PO₃M,-O-PO₃M₂ or -SO₃M.

In formula (II), R¹ may be a straight chain or branched C₁₋₁₆ alkyl group, preferably a straight chain or branched C₁₋₁₆ alkyl group. Examples of the C₁₋₁₆ alkyl group of R¹ are methyl, ethyl, n-propyl, i-propyl, n-butyl, isobutyl, tert-butyl, sec-butyl, pentyl and hexyl. An aryl group may, for example, be a phenyl group or a naphthyl group. A C₃₋₁₄ heteroaryl group may contain 1 to 3 heteroatoms selected from nitrogen, oxygen and sulfur.

According to a preferred embodiment, R¹ is a hydrogen atom. In case R¹ is a C₁₋₆ alkyl group, the C₁₋₆ alkyl group is preferably substituted by -COOM.

In formula (II), R² is a hydrogen atom, -COOM, a straight chain or branched C₁₋₁₆ alkyl group which may be substituted by a C₆₋₁₄ aryl or C₃₋₁₄ heteroaryl group, -COOM, -PO₃M, -O-PO₃M₂ and -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, or a C₆₋₁₄ aryl or C₃₋₁₄ heteroaryl group which may be substituted by -COOM, -PO₃M, -O-PO₃M₂ and -SO₃M.

In formula (II), R² may be a straight chain or branched C₁₋₁₆ alkyl group, preferably a straight chain or branched C₁₋₁₆ alkyl group. Examples of the C₁₋₁₆ alkyl group of R² methyl, ethyl, n-propyl, i-propyl, n-butyl, isobutyl, tert-butyl sec-butyl, pentyl and hexyl. An aryl group may, for example, be a phenyl group or a naphthyl group. A C₃₋₁₄ heteroaryl group may contain 1 to 3 heteroatoms selected from nitrogen, oxygen and sulfur.

According to a preferred embodiment, R² is a hydrogen atom or a methyl group.

The following groups are preferred groups of formula (II), wherein M is a hydrogen atom or a metal atom:

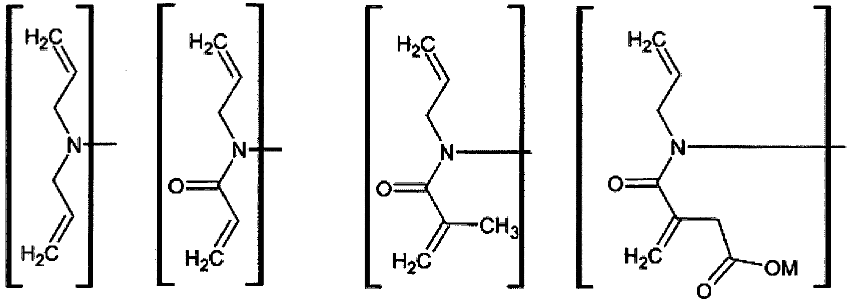

In formula (I), L is a single bond or an (n+1) -valent linker group.

The linker group has a valency of at least two which corresponds to the total number of substituents A and B. Accordingly, L may be preferably divalent (n=2), trivalent (n=3), tetravalent (n=4), or pentavalent (n=5). Preferable, when L is a linker group, L is divalent or trivalent, most preferably divalent.

In general, when L is a linker group, L is a linear or branched monomeric, oligomeric, polymeric or copolymeric group. A monomeric group is a low-molecular group having a molecular weight of up to 500. An oligomeric group is a group having a molecular weight of more than 500 to up to 10000 and may be prepared by a polymerization reaction. A polymeric or copolymeric group is a group having a molecular weight of more than 10000 which may be obtained by a polymerization reaction. The polymerization may be a condensation or addition reaction. The reation may be a ring-opening polymerization.

The linker group L may be a hydrocarbon group which may be aliphatic and/or aromatic. The hydrocarbon group may be substituted by 1 to 6 C₁₋₄ alkyl groups. Specific examples of the alkyl groups are methyl, ethyl, n-propyl, i-propyl, n-butyl, i-butyl or tert.-butyl. In a preferred embodiment, the hydrocarbon group of the linker group L may contain 1 to 5 oxygen atoms in the hydrocarbon group in the form of aliphatic or aromatic ether bonds, keto groups, carboxylic acid groups, or hydroxyl groups. Ester groups are not preferred in moiety L in view of hydrolysis stability of the polymerizable monomer. In case of an aliphatic group, L may be a straight chain or branched chain alkylene group or a cycloalkylene group. In case of an aromatic group, A may be an arylene group or a C₃₋₁₄ heteroarylene group. Specifically, L may be a divalent substituted or unsubstituted C₁ to C₂₀ alkylene group, substituted or unsubstituted C₆₋₁₄ arylene group, substituted or unsubstituted C₃ to C₂₀ cycloalkylene group, substituted or unsubstituted C₇ to C₂₀ arylenealkylenearylene group.

According to a preferred embodiment, L represents a saturated aliphatic C₂₋₂₀ hydrocarbon chain which may contain 2 to 4 oxygen atoms or nitrogen atoms, and which may be substituted by 1 to 6 C₁₋₄ alkyl groups, or L may be a substituted or unsubstituted C₇ to C₂₀ arylenealkylenearylene group which may be substituted by 1 to 6 C₁₋₄ alkyl groups.

In case L is an (n+1) -valent linker group, the linker group may preferably be a C₁₋₁₂ hydrocarbon group. The C₁₋₁₂ hydrocarbon group may contain 1 to 3 carbonyl groups or heteroatoms selected from oxygen, nitrogen and sulfur. Moreover, the C₁₋₁₂ hydrocarbon group may be substituted by a hydroxyl group, a C₆₋₁₄ aryl group, -COOM, -PO₃M, -O-PO₃M₂ or -SO₃M, wherein M is a hydrogen atom or a metal atom. Specific examples of a divalent C₁₋₁₂ hydrocarbon group are a straight chain or branched C₁₋₁₂ alkylene group such as a methylene, ethylene, propylene, butylene or phenylene group which groups may be substituted by a hydroxyl group, a C₆₋₁₄ aryl group, -COOM, -PO₃M, -O-PO₃M₂ or -SO₃M.

In case L is an divalent linker group, the linker group may be a polyamide residue of the following formula (V)

-Y-L'-Y'- (V)

wherein
Y and Y' represent CO and the polyamide moiety of formula (V) is obtainable by a process comprising the step of a step-growth polymerization of a mixture containing a diamine of the following formula (VI) and a compound of the following formula (VII) having at least two carboxylic acid groups,

R³(NHR")_{y} (VI)

wherein
- R³: represents an y-valent C₂₋₂₀ straight-chain, branched or cyclic hydrocarbon group which may optionally contain from 1 to 6 hetero atoms selected from nitrogen, oxygen, or sulphur atoms in the backbone of the hydrocarbon group, and optionally from 1 to 6 functional groups selected from hydroxyl groups, thiol groups and amino groups;
- R": represents a hydrogen atom, an allyl group,
a straight chain or branched C₁₋₆ alkyl group which may be substituted by a C₃₋₆ cycloalkyl group, or a C₆₋₁₄ aryl group,
a C₃₋₆ cycloalkyl group which may be substituted by a C₁₋₆ alkyl group or a C₆₋₁₄ aryl group; and
- y: represents an integer of at least 2;

MOOC-R⁴-COOM (VII)

wherein R⁴ represents a C₁₋₂₀ straight-chain, branched, cyclic or aromatic hydrocarbon group which may optionally contain a carbon-carbon double bond, from 1 to 6 heteroatoms selected from nitrogen, oxygen, or sulphur atoms in the backbone of the hydrocarbon group, and optionally from 1 to 6 functional groups selected from carboxylic acid groups, hydroxyl groups, thiol groups and amino groups, and the M which may be the same or different, independently represent a hydrogen atom or a metal atom.

Preferred divalent linker groups may be selected from methylene, ethylene, propylene, butylene and the following divalent groups:

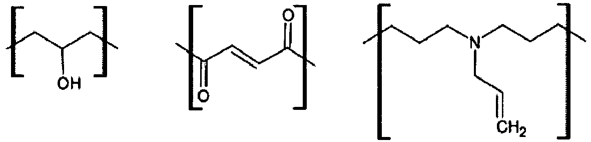

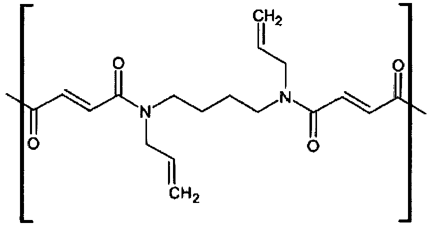

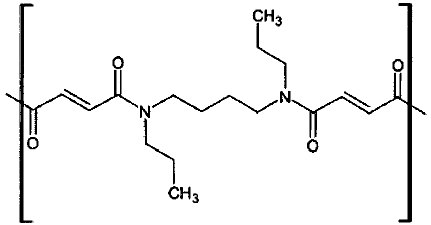

In formula (I), B independently is a group according to the definition of A, a group of the following formula (III), a group of the following formula (IV), or a group of the following formula [Z'X"]ₘE.

When B is a group according to formula (II) in the definition of A, then B may be the same or different from the group A present in a polymerizable compound of formula (I). Moreover, in case more than one group B according to the definition of A is present in the polymerizable compound of formula (I), the groups B may be the same or different. Specifically, B may be a group of the above formula (II) wherein an allyl group is bonded to a nitrogen atom, and a further group having a polymerizable double bond is bonded to the same nitrogen atom.

When B is a group according to formula (II) in the definition of A, X¹ is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10.

When B is a group according to formula (II) in the definition of A and in case X¹ is a group [X'Z]ₖ), it is preferred that L provides a polymerizable double bond which may take part with the allyl group directly bonded to the nitrogen atom of the group of formula (II) in a cyclopolymerization reaction. Preferably, X' is an oxygen atom. Preferred Examples for a straight chain or branched C₁₋₄ alkylene group for Z are an ethylene group and a propylene group. Preferably, k is an integer of from 1 to 4.

According to a preferred embodiment of B being a group according to formula (II) in the definition of A, X¹ is CO.

When B is a group according to formula (II) in the definition of A, R¹ is a hydrogen atom,-COOM, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or-SO₃M, a C₃₋₆ cycloalkyl group which may be substituted by a C₁₋₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M.

According to a preferred embodiment, R¹ is a hydrogen atom. In case R¹ is a C₁₋₆ alkyl group, the C₁₋₆ alkyl group is preferably substituted by -COOM.

When B is a group according to formula (II) in the definition of A, R² is a hydrogen atom,-COOM, a straight chain or branched C₁₋₁₆ alkyl group which may be substituted by a C₆₋₁₄ aryl group, -COOM, -PO₃M, -O-PO₃M₂ and -SO₃M, a C₃₋₆ cycloalkyl group which may be substituted by a C₁₋₁₆ alkyl group, a C₆₋₁₄ aryl group, -COOM, -PO₃M, -O-PO₃M₂ or -SO₃M, or a C₆₋₁₄ aryl or C₃₋₁₄ heteroaryl group which may be substituted by -COOM, -PO₃M, -O-PO₃M₂ and -SO₃M,

According to a preferred embodiment of B being a group according to formula (II) in the definition of A, R² is a hydrogen atom or a methyl group.

When B is a group of the formula (III), B is as follows.

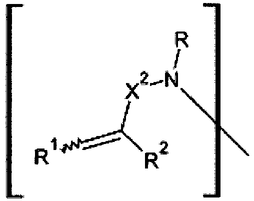

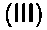

In formula (III), X² has the same meaning as defined for X¹ in formula (II). Moreover, in case more than one group X² according to the definition of X¹ is present in the polymerizable compound of formula (I), the groups X² may be the same or different. Specifically, When B is a group according to formula (III), X² is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10. X', Z and m of a group of formula (III) maybe the same or different of X', Z and k of any group of formula (II) present as A or B in the polymerizable compound of fromula (I).

When B is a group according to formula (III) and in case X² is a group [X'Z]ₖ), it is preferred that L provides a polymerizable double bond which may take part with the allyl group directly bonded to the nitrogen atom of the group of formula (II) in a cyclopolymerization reaction. Preferably, X' is an oxygen atom. Preferred Examples for a straight chain or branched C₁₋₄ alkylene group for Z are an ethylene group and a propylene group. Preferably, k is an integer of from 1 to 4.

According to a preferred embodiment of B being a group according to formula (III), X¹ is CO.

In formula (III), R¹ and R² are independent from each other and independently have the same meaning as defined for formula (II). Moreover, in case more than one group R¹ and R², respectively, is present when more than one group B of formula (III) is present in the polymerizable compound of formula (I), each of the groups R¹ and R² may be the same or different, respectively.

Specifically, when B is a group according to formula (III), R¹ is a hydrogen atom, -COOM, a straight chain or branched C₁₋₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M.

According to a preferred embodiment when B is a group according to formula (III), R¹ is a hydrogen atom. In case R¹ is a C₁₋₁₆ alkyl group, the C₁₋₆ alkyl group is preferably substituted by -COOM.

When B is a group according to formula (III), R² is a hydrogen atom, -COOM, a straight chain or branched C₁₋₆ alkyl group which may be substituted by a C₆₋₁₄ aryl or C₃₋₁₄ heteroaryl group, -COOM, -PO₃M, -O-PO₃M₂ and -SO₃M, a C₃₋₆ cycloalkyl group which may be substituted by a C₁₋₆ alkyl group, a C₆₋₁₄ aryl or C₃₋₁₄ heteroaryl group, -COOM, -PO₃M, - O-PO₃M₂ or -SO₃M, or a C₆₋₁₄ aryl or C₃₋₁₄ heteroaryl group which may be substituted by - COOM, -PO₃M, -O-PO₃M₂ and -SO₃M,

According to a preferred embodiment of B being a group according to formula (III), R² is a hydrogen atom or a methyl group.

In formula (III), R is a hydrogen atom, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl group which may be substituted by -COOM, -PO₃M, -O-PO₃M₂ or -SO₃M.

Preferred groups of B of the formula (III) are as follows wherein M is a hydrogen atom o a metal atom.

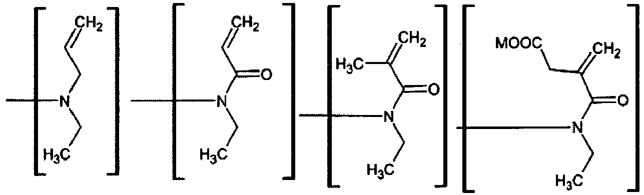

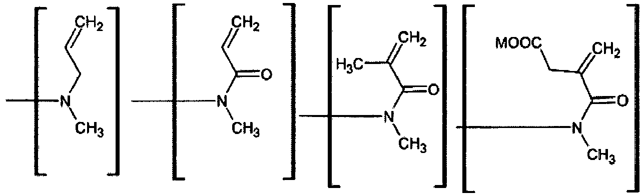

When B is a group of the formula (IV), B is as follows.

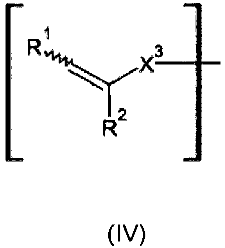

In formula (IV), X³ is CO, -CH₂CO-, CS, or -CH₂CS-,

According to a preferred embodiment, X³ is CO, -CH₂CO-.

In formula (IV), R¹ and R² are independent from each other and independently have the same meaning as defined for formula (II). Moreover, in case more than one group R¹ and R², respectively, is present when more than one group B of formula (IV) is present in the polymerizable compound of formula (I), each of the groups R¹ and R² may be the same or different, respectively.

Specifically, when B is a group according to formula (IV), R¹ is a hydrogen atom, -COOM, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M.

According to a preferred embodiment when B is a group according to formula (IV), R¹ is a hydrogen atom. In case R¹ is a C₁₋₁₆ alkyl group, the C₁₋₁₆ alkyl group is preferably substituted by -COOM.

When B is a group according to formula (IV), R² is a hydrogen atom, -COOM, a straight chain or branched C₁₋₁₆ alkyl group which may be substituted by a C₆₋₁₄ aryl or C₃₋₁₄ heteroaryl group, -COOM, -PO₃M, -O-PO₃M₂ and -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, - O-PO₃M₂ or -SO₃M, or a C₆₋₁₄ aryl group which may be substituted by -COOM, -PO₃M, -O-PO₃M₂ and -SO₃M,

According to a preferred embodiment of B being a group according to formula (IV), R² is a hydrogen atom or a methyl group.

Preferred groups of formula (IV) are as follows, whereby M is a hydrogen atom or a metal atom.

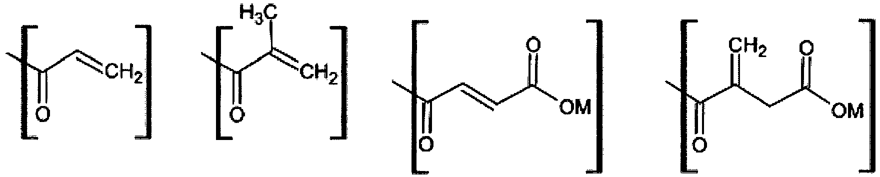

When B is a group [Z'X"]ₘE, the meaning of Z', X", m, and E is as follows. Z' is a straight chain or branched C₁₋₄ alkylene group. Specific examples of the C₁₋₄ alkylene groups are methylene, ethylene, propylene and butylene.

X" is an oxygen atom, a sulfur atom or NH.

E is a hydrogen atom, PO₃M₂, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or-SO₃M.

m is an integer of from 1 to 10.

In formula (I), n is an integer of from from 1 to 4, preferably an integer of from 1 to 3. According to a first preferred generic group of compounds of formula (I), n is 1. According to a second preferred generic group of compounds of formula (I), n is 2.

According to the definition of the present invention, the M which are independent from each other, each represent a hydrogen atom or a metal atom. The metal atom is preferably an alkali metal or an alkaline earth metal. Specific examples of the alkali metal are lithium, sodium, and potassium. Specific examples of the alkaline earth metal are calcium, strontium and magnesium. The metal atom may also be tin.

According to a specific embodiment, the compound of formula (I) is not a (meth)acrylamide containing only a single (meth)acryl group such as in the case of N,N-diallyl acrylamide, but a compound containing at least two (meth)acryl groups.

The polymerizable compound of formula (I) may be prepared according to the following scheme, wherein A represent polymerizable moieties, B and C represent moieties which may form a linkage B-C. The linkage B-C may be formed by a condensation reaction or an addition reaction. The reaction may involve the formation of an amide bond, a urethane bond, and an ester bond. Preferably, the reaction involves the formation of an amide bond or a urethane bond. 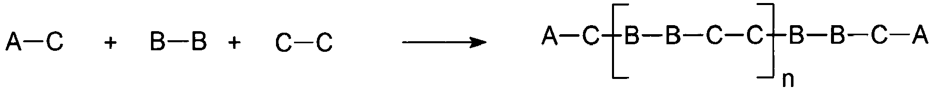 The preparation reaction may be carried out in a mixture of a compound having a single moiety C, a compound having two moieties B and a compound having two moieties C. Accordingly, a chain distribution of (B-B-C-C)n is observed whereby the certain positions such as the terminal positions, may be capped by a moiety A. Alternatively, it is possible to prepare a polymerizable compound of formula (I) by a reaction wherein a stoichiometric mixture of compound A-C and B-B is reacted and then the reaction product is reacted with a compound C-C. Alternatively, it is possible to prepare a polymerizable compound of formula (I) by a reaction wherein a mixture of compound B-B and C-C is reacted and then the reaction product is end-capped with A-C.

A specific example is as follows.

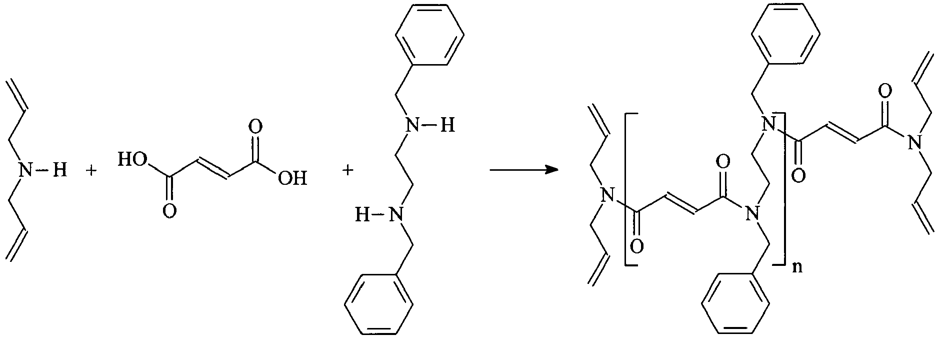

The reaction may be performed in accordance with, for example, the methods described in Jerry March "Advanced Organic Chemistry" Fourth Edition, John Wiley & Sons, INC., 1992 Richard C. Larock "Comprehensive Organic Transformation", VCH Publishers, INC., 1989 or a method conforming to the methods described above. The reaction may be carried out in a solvent which is capable of dissolving the reactants. An example of a suitable solvent is toluene. The reaction tempeature is not particularly limited. A suitable reaction temperature is from -30 °C to the boiling point of the solvent. The reaction time is not particularly limited and may be selected from 5 minutes to 48 hours.

### Initiator

The dental composition according to the present invention may comprise a polymerization initiator system. The initiator system may be based on a redox initiator or on a photoinitiator.

In case the dental composition contains a redox initiator, the amount of reducing agent and oxidizing agent should be sufficient to provide the desired degree of polymerization. Preferably, the mixed but unset cements of the invention contain a combined weight of about 0.01 to about 10%, more preferably about 0.2 to about 5%, and most preferably about 0.3 to about 3% of the reducing agent and oxidizing agent, based on the total weight (including water) of the mixed but unset cement components. The reducing agent or the oxidizing agent can be microencapsulated as described in U.S. Pat. No. 5,154,762. This will generally enhance shelf stability of the cement parts and if necessary permit packaging both the reducing agent and oxidizing agent together. Water-soluble and water-insoluble encapsulants can be employed. Suitable encapsulating materials include cellulosic materials as cellulose acetate, cellulose acetate butyrate, ethyl cellulose, hydroxymethyl cellulose and hydroxyethyl cellulose being preferred. Other encapsulants include polystyrene, copolymers of polystyrene with other vinylic monomers and polymethylmethacrylate, copolymers of methylmethacrylate with other ethylenically-unsaturated monomers. Preferred encapsulants are ethylcellulose and cellulose acetate butyrate. By varying the choice of encapsulant and the encapsulation conditions, the onset of curing can be tailored to start at times ranging from seconds to minutes. The ratio of amount of encapsulant to activator generally ranges from 0.5 to about 10 and preferably from about 2 to about 6.

Suitable oxidizing agents (initiators) include peroxides such as benzoyl peroxide, cumene hydroperoxide (CHP), and tert-butyl hydroperoxide, ferric chloride, hydroxylamine (depending upon the choice of reducing agent), perboric acid and its salts, and salts of a permanganate or persulfate anion. Preferred oxidizing agents are peroxides, potassium persulfate, ammonium persulfate and hydrogen peroxide.

Suitable reducing agents (activators) include ascorbic acid, benzyl thiourea ferrous chloride, ferrous sulfate, hydrazine, hydroxylamine (depending upon the choice of oxidizing agent) oxalic acid, thiourea, and salts of a dithionite or sulfite anion. Preferred reducing agents include ascorbic acid and ferrous sulfate.

A photoinitiator should be capable of promoting polymerization of the polymerizable groups on exposure to light of a suitable wavelength and intensity. The photoinitiator preferably is sufficiently shelf-stable and free of undesirable coloration to permit its storage and use under typical dental conditions. Visible light photoinitiators are preferred. Suitable visible light-induced and ultraviolet light-induced initiators include an alpha-diketone (e.g., camphorquinone) with or without additional hydrogen donors (such as sodium benzene sulfinate, amines and amine alcohols). The photoinitiator may be present in an amount sufficient to provide the desired rate of photopolymerization. This amount will be dependent in part on the light source, the thickness of the cement layer to be exposed to radiant energy and the extinction coefficient of the photoinitiator. Preferably, mixed but unset photocurable cements of the invention will contain about 0.01 to about 5%, more preferably from about 0.1 to about 2% photoinitiator, based on the total weight (including water) of the mixed but unset cement components.

### Free radical polymerizable monomers

The dental composition according to the present invention may further contain one or more free radical-polymerizable monomers. Preferable are (meth)acrylate monomers and corresponding (meth)acrylamide monomers which are generally used for dental materials. Examples of such monomers include linear, branched or cyclic omega-methacryloyloxy(C₂₋₁₈)alkyl dihydrogen phosphate, in particular 10-methacryloyloxydecyl dihydrogen phosphate (MDP), 2-hydroxyethyl (meth)acrylate, N,N'-diethyl-1,3-propylene bisacrylamide, ethyl-2-[5-dihydrogenphosporyl-5,2-dioxapentyl] acrylate, ethylene glycol di(meth)acrylate, neopentyl glycol di(meth)acrylate, triethylene glycol di(meth)acrylate, 2,2-bis[4-(3-methacryloyloxy-2-hydroxypropoxy)phenyl]propane (abbreviated as Bis-GMA), 2,2-bis[(meth)acryloyoxypolyethoxyphenyl]propane, trimethylolpropane tri(meth)acrylate, (meth)acrylic acid, 2-methacryloyloxyethyl dihydrogen phosphate , bis[2-methacryloyloxyethyl] hydrogen phosphate, 4-(2-methacryloyloxyethoxycarbonyl]phthalic acid anhydride (popular name: 4-META), 4-[2-methacryloyloxyethoxycarbonyl]phthalic acid (popular name: 4-MET), 11,11-dicarboxyundecyl(meth)acrylate, 3-methacryloyloxypropyltrimethoxysilane, 2-(N,N-dimethylamino)ethylmethacrylate, and the like. According to a preferred embodiment, the dental composition may further contain 10-methacryloyloxydecyl dihydrogen phosphate (MDP).

The free radical-polymerizable monomers may be contained in the dental composition of the present invention in an amount of from 0.1 to 50 percent, peferably 1.0 to 40 percent by weight based on the total weight of the composition.

### Particulate filler

In case the dental composition of the present invention is a dental adhesive composition, a bonding agent or a dental desensitizing composition, the dental composition may further contain a particulate filler. The particulate filler may have an average particle size in the range of from 1 to 100 nm, preferably in the range of from 3 to 60 nm. The particulate filler may be present in an amount of from 0.05 to 20 percent by weight based on the total weight of the dental composition.

In case the dental composition of the present invention is a pit and fissure sealant, a pulp capping composition, a dental composite, dental glass ionomer cement, a dental cement or a dental root canal sealer composition, the dental composition may further contain a particulate filler. The particulate filler may have an average particle size in the range of from 1 nm to 100 µm, preferably in the range of from 1 to 50 µm. The particulate filler may be present in an amount of from 0.5 to 80 percent by weight based on the total weight of the dental composition.

The invention will now be further explained based on the following examples:

### Example 1

### Preparation of 1,3-Bis-allylamino-2-propanol dihydrochloride salt

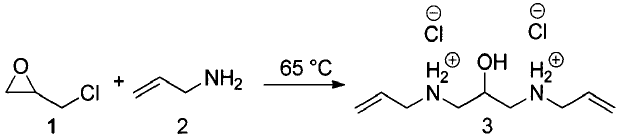

A 250 mL three-necked flask, equipped with septum and condenser, was charged with 75 mL allyl amine (1000 mmol) and was heated to 65 °C (oil bath temperature). Then, with continuous stirring, 19,6 mL epichlorohydrin (250 mmol) were added dropwise over four hours and the mixture was stirred further for 16 hours under reflux. The white solid product was obtained by precipitation in acetone and has been washed with acetone to remove remaining byproducts and starting materials. The compound was evaporated under vacuum to dryness.

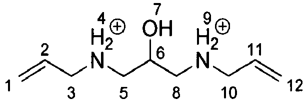

| | |
|---|---|
| **Yield:** | 14,5 g (24 % d.Th.) |
| **IR v [cm⁻¹]:** | 3290 (m), 3089 (w), 2947 (s), 2719 (s), 2431 (s), 1648 (w), 1447 (s), 1427 (m), 1113 (w), 992 (s), 931 (s) |
| **¹H-NMR [ppm]:** | (300 MHz, CDCl₃): 2.87-3.09 (m, 4H, H 5, 8), 3.57 (d, 4H, ³J_{H}= 6.82 Hz, H 3, 10), 4.27-4.31 (m, 1H, H 6), 5.38 (dd, 2H, ⁴J_{H}= 1.41 Hz, ³J_{H}= 10,39 Hz, H 1, 12), 5.47 (dd, 2H, ⁴J_{H}= 1.41 Hz, ³J_{H}= 17.19 Hz, H 1, 12), 5.87-6.00 (m, 2H, H 2, 11), 9,41 (s, 4 H, H 4, 9) |
| **MS (EI):** | m/z = 171 |

### 1.) Acrylation of 1,3-Bis-allylamino-2-propanol dihydrochloride salt

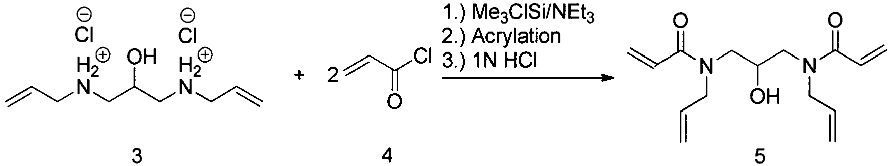

A 250 mL three-necked flask, equipped with septum and condenser, was charged with 2225 mg 1,3-Bis-allylamino-2-propanol dihydrochloride (9,18 mmol) solved in 100 mL Dichloromethane and 7,63 mL Triethylamine (101,2 mmol) under nitrogene atmosphere. The mixture was cooled to 5 °C and 4,1 mL Chlorotrimethylsilane (32,13 mmol) was added dropwise. After complete addition the reaction stirred overnight. Then, 1,6 mL Acryloyl chloride (20,2 mmol) solved in 20 mL dichloromethane was added dropwise keeping the temperature at -78 °C and stirred at room temperature for about 4 hours. After that, 100 mL 1N HCl was added and stirred for 2 hours. The organic layer was washed with 100 mL 1N HCl three times, dried over MgSO₄, filtered and evaporated under vacuum to dryness. The residue was purified by column chromatography eluting with CH₂Cl₂/EtOH (volume ratio, 95:5) to give a viscous liquid from which a yellowish solid cristallizes.

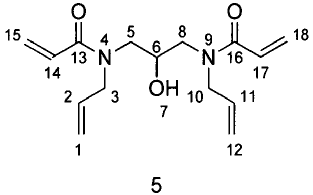

| | |
|---|---|
| **Yield:** | 1,52 g (59 % d.Th.) |
| **IR v [cm⁻¹]:** | 3370 (m), 3084 (w), 2927 (m), 1641 (s), 1605 (s), 1442 (s), 1417 (s), 1223 (s), 1101 (m), 795 (s) |
| **¹H-NMR [ppm]:** | (600 MHz, CDCl₃): 3.40-3.48 (m, 4H, H 5, 8), 4.04-4.07 (m, 4H, H 3, 10), 4.72 (d, 1H, H 6), 5.09 (d, 2H, H 1, 12), 5.16 (d, 2H, H 1, 12), 5.64 (d, 2H, H 15, 18), 5.70-5.78 (m, 2H, H 2,11), 6.29 (d, 2H, H 15, 18), 6.42 (dd, 2H, H 14, 17) |
| **¹³C-NMR [ppm]:** | (150 MHz, CDCl₃): 51.90 (C 3, 10), 51.93 (C 5, 8), 71.80 (C 6), 117.07 (C 1, 12), 127.46 (C 2, 11), 129.00 (C 15, 18), 132.76 (C 14, 17), 168.55 (C 13, 16) |

The heat of polymerization of the compound of Example 1 is as follows:
D_{R}H= -129.7 ± 1.1 kJ/mol (37°C),
D_{R}H= -190.4 ± 21.0 kJ/mol (80°C), (p= 68.1%)
Considering that an acrylate group contributes to the heat of polymerization with about 80 kJ/mol, a compound of Example 1 would be expected to show a heat of polymerization of about 109 kJ/mol due to the presence of two acrylate groups reacting at a rate of 68.1%. Accordingly, the remaining heat of polymerization of about 81 kJ/mol would be due to the allyl group. Given that usually, the allyl group does not take part in a radical polymerization, and given that in the present case each allyl group would contribute at a rate of 40.5 kJ/mol, a compound of the present invention provides reactive allyl groups which appear to contribute at a rate of 60 kJ/mol which is similar to a methacrylate group.

### Example 2 (not according to the invention)

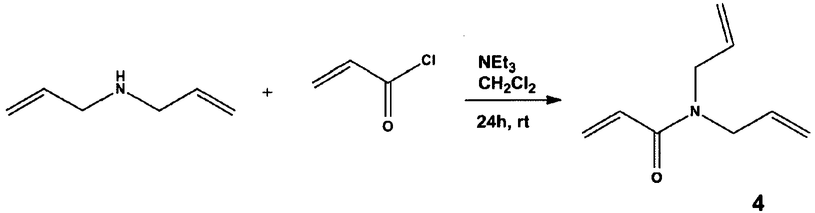

Compound **4** has been prepared by addition of acryloyl chloride and triethylamine to diallylamine in dichloromethane after stirring the mixture at room temperature for 24 hours. After vacuum distillation the product could be obtained in excellent purity, however, the yield of the reaction was 23 %.

After work-up by column chromatography (n-hexane : ethyl acetate, 90 : 10 to 75 : 25) 79 % of compound **1** could be obtained.
**¹H-NMR** (600 MHz, CDCl₃): *δ* [*ppm*] = 3.94 (*dd,* 2H, *J* = 4.4, 2.1 Hz, 3a-H), 4.04 (*dt,* 2H, *J* = 6.1, 1.4 Hz, 3b-H), 5.12-5.24 (*m,* 4H, 5a,b-H), 5.68 (*dd*, 1H, *J* = 10.4, 2.1 Hz, 1a-H), 5.75-5.83 (*m,* 2H, 4a,b-H), 6.37 (*dd*, 1H, *J* = 16.8, 2.1 Hz, 1b-H), 6.49 (*dd*, 1H, *J* = 16.7, 10.3 Hz, 2-H).
IR v̂_{max□} [*cm⁻¹*] = 3082 (w), 3012 (w), 2984 (w) 2916 (w), 1848 (w), 1649 (s, Amide I), 1613 (s), 1464 (m), 1437 (s), 1415 (s), 1360 (w), 1343 (w), 1277 (w), 1221 (s), 1193 (m), 1138 (w), 1059 (w), 979 (m), 956 (m), 920 (s), 794 (s), 686 (w), 653 (w), 558 (m), 508 (w).
**GC-MS** *^{m}*/*_{z}* (*l* in %) = 151 (8), 110 (25), 70 (25), 56 (55), 55 (100), 41 (25).

The heat of polymerization of the compound of Example 2 is as follows:
D_{R}H(37°C) = -135.1 ± 4.0 kJ/mol

The heat of polymerization indicates a reactivity of the allyl groups in a radical polymerization which is comparable to an acrylate group.

### Example 3

### N,N' -Diallylpropane-1 ,3-diamine

Reference: Anita H. Lewin et al., J. Med. Chem., 1998, 41 (6), pp 988-995

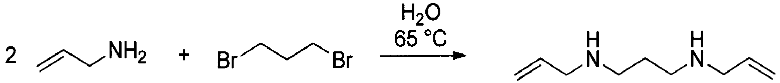

To a cold (0 °C) mixture of 4.06 mL 1,3-dibromopropane (40 mmol) and 2.2 mL H₂O was added 15 mL allylamine (200 mmol). The mixture was slowly allowed to warm to room temperature and then heated to reflux overnight. The resulting solution was diluted with a small portion of H₂O and saturated with solid KOH. The mixture was then extracted with EtOAc (3 x 60 mL), dried over MgSO₄ and evaporated under vacuum.

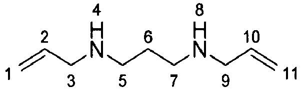

| | |
|---|---|
| **Yield:** | 4.7 g (77 %) |
| **IR v [cm⁻¹]:** | 3272 (w, br), 3075 (m), 3006 (w), 2977 (w), 2923 (m), 2806 (s), 1738 (m), 1642 (m), 1453 (s), 1417 (m), 1241 (m), 1116 (s), 993 (s), 913 (s), 748 (s), 634 (m) |
| **¹H-NMR [ppm]:** | (300 MHz, CDCl₃): 1.70 (tt, 2H, ³J_{H}= 6.8 Hz, **H 6**), 2.69 (t, 4H, ³J_{H}= 6.8 Hz, **H 5, 7**), 3.23 (dt, 4H, ³J_{H}= 6.0 Hz, ⁴J_{H}= 1.4 Hz, **H 3, 9**), 5.05-5.10 (m, 2H, **H 1, 11**), 5.11-5.19 (m, 2H, **H 1, 11**), 5.80-5.94 (m, 2H, **H 2, 10**) |
| **¹³C-NMR [ppm]:** | (75 MHz, CDCl₃): 29.84 (**C 6**), 47.97 (**C 5, 7**), 52.38 (**C 3, 9**), 116.21 (**C 1, 11**), 136.48 (**C 2, 10**) |

### N,N-Di(allyl acrylamido) propane

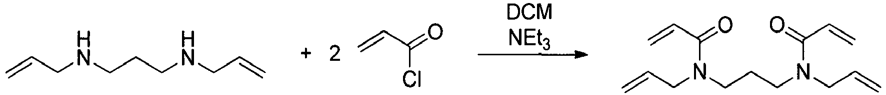

A 500 mL three-necked flask, equipped with septum and condenser, was charged with 2.25 g N,N'-diallylpropan-1,3-diamine (14.6 mmol) solved in 250 mL dichloromethane and 12.1 mL triethylamine (88 mmol) under nitrogen atmosphere. Then, 5.9 mL acryloyl chloride (73 mmol) dissolved in 20 mL dichloromethane was added dropwise keeping the temperature at -78 °C and stirred at room temperature overnight. After that, 100 mL 1N HCl was added and stirred for 2 hours. The layers were separated and the aqueous layer was washed with 100 mL dichloromethane. After that, the combined organics were washed with 100 mL 1N HCl three times, dried over MgSO₄, filtered and evaporated under vacuum to dryness. The residue was purified by flash column chromatography eluting with CH₂Cl₂/EtOH (volume ratio, 95:5) to give a light yellow liquid. The product is moderately soluble in water.

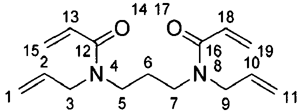

| | |
|---|---|
| **Yield:** | 1.0 g (27%) |
| **IR v [cm¹]:** | 3491 (w,br), 3080 (w), 3011 (w), 2979 (w), 2931 (w), 1721 (w), 1644 (s), 1609 (s), 1427 (s), 1376 (m), 1320 (w), 1272 (w), 1218 (s), 1134 (m), 1093 (w), 1057 (m), 977 (s), 921 (s), 859 (w), 794 (s) |
| **¹H-NMR [ppm]:** | (300 MHz, CDCl₃): 1.83 (tt, 2H, ³J_{H}= 7.5 Hz, **H 6**), 3.30-3.41 (m, 4H, **H 5**, **7**), 3.95-4.02 (m, 4H, **H 3, 9**), 5.09-5.22 (m, 4H, **H 1**, **11**), 5.61-5.83 (m, 4 H, **H 2, 10, 15, 19),** 6.25 (m, 4H, **H 13, 15, 18, 19**) |
| **¹³C-NMR [ppm]:** | (75 MHz, CDCl₃): 27.88 (**C 6**), 44.36 (**C 5, 7**), 50.21 (**C 3, 9**), 116.93 (**C 1, 11**), 128.05 (**C 13, 15, 18, 19**), 133.10 (**C 2, 10**), 166.72 (**C 12**, **16**) |

### Example 4

### Synthesis of N-allylacrylamide

2.28 g of allylamine (40 mmol) were stirred in 40 ml of dichloromethane under cooling. 4.05 g of triethylamine (40 mmol) in 15 ml of dichloromethane were added to this solution. Afterwards, 1.5 eq (60 mmol, 5.43 g) of acryloyl chloride in 15 ml of dichloromethane were added dropwise under cooling. The resulting mixture was stirred for 24 hours at room temperature. Then the solution was extracted three times with 100 ml of water. The organic layer was dried over sodium sulfate and concentrated under reduced pressure.

The obtained crude product, a colourless liquid, was then purified by vacuum distillation (bp 58-59°C, 2.6 ·10-2 mbar). Yield: 28 %

### Example 5

### (2E)-N,N'-di(prop-2-en-1-yl)but-2-ene-1,4-diamine

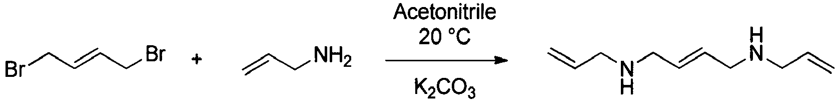

A 500 mL flask, equipped with a dropping funnel, was charged with 145 mL allylamine (1930 mmol), 100 mL acetonitrile and 33.3 g potassium carbonate (241 mmol). 20.6 g trans-1,4-dibromo-2-butene (96.5 mmol) dissolved in 100 mL acetonitrile was added dropwise over one hour and the solution was stirred for one hour. Solids were filtered off and the excess of allylamine and acetonitrile were evaporated under vacuum to give a colorless liquid. The product was used in next steps without further purification.

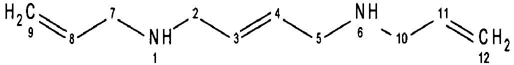

| | |
|---|---|
| **Yield:** | 12.5 g (78 % d.Th.) |
| **IR v [cm-¹]:** | 3249 (m, br), 3075 (m), 2976 (m), 2916 (m), 2810 (m), 1641 (s), 1488 (w), 1450 (m), 1418 (m), 1364 (w), 1200 (w), 1117 (m), 992 (s), 972 (s), 913 (s), 744 (s), 634 (w), 562 (w). |
| **¹H-NMR [ppm]:** | (300 MHz, CDCl₃): 1.08 (s, **H 1, 6**), 3.20-3.24 (m, 8H, **H 2, 5, 7, 10**), 5.06 (dq, 2H, J = 1.4 Hz, J = 10.2 Hz, **H 9,12**), 5.14 (dq, 2H, J = 1.7 Hz, J = 17.1 Hz, **H** 9, **12**), 5.67 (m, 2H, **H 3, 4**), 5.81-5.94 (m, 2H, **H 8, 11**). |

### N,N'-(2E)-but-2-en-1,4-diallylbis-[(N-prop-2-en-1) amide

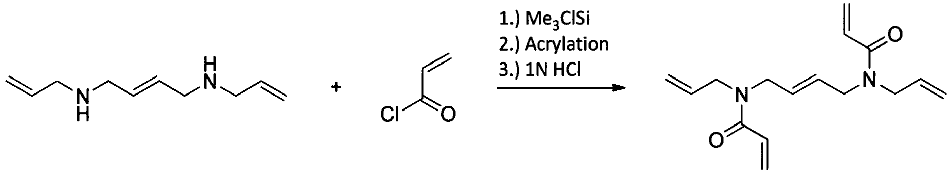

A 2 L three-necked flask, equipped with dropping funnel, KPG-stirrer and condenser, was charged with 12 g (2E)-N,N'-di(prop-2-en-1-yl)but-2-ene-1,4-diamine (72.2 mmol) dissolved in 500 mL dichloromethane, 65 mL triethylamine (469 mmol) and 10 mg 2,6-di-tert-butyl-4-methylphenol (BHT) under nitrogen atmosphere. The mixture was cooled to ∼5 °C, 23 mL chlorotrimethylsilane (180 mmol) was added dropwise and stirred for 30 minutes. Then, 29.2 mL acryloyl chloride (361 mmol) dissolved in 150 mL dichloromethane was added dropwise keeping the temperature below -50 °C and stirred at room temperature for 90 minutes. After that, 200 mL 1N HCl was added and stirred for 30 minutes. The layers were separated and the organic layer was washed with 200 mL 1N HCl two times, dried over MgSO₄, filtered and evaporated under vacuum to dryness. The residue was purified by flash column chromatography eluting with CH₂Cl₂/EtOH (volume ratio, 95:5) to give a light yellow liquid.

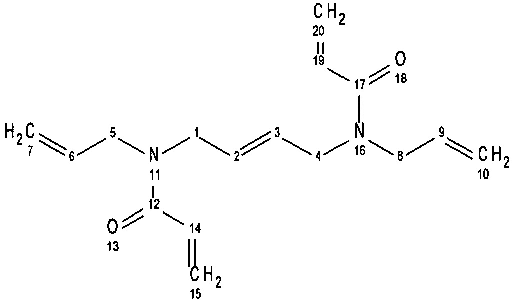

| | |
|---|---|
| **Yield:** | 10.3 g (52 % d.Th.) |
| **IR v [cm⁻¹]:** | 3080 (w), 3011 (w), 2974 (w), 2912 (w), 1645 (s), 1610 (s), 1463 (m), 1437 (s), 1416 (s), 1361 (m), 1309 (w), 1275 (w), 1263 (w), 1214 (s), 1189 (m), 1131 (w), 1063 (w), 1027 (w), 975 (s), 925 (s), 794 (s), 732 (m), 697 (w), 668 (m), 630 (m), 557 (m). |
| **¹H-NMR [ppm]:** | (300 MHz, CDCl₃): 3.88-4.08 (m, 8H, **H 1, 4, 5, 8**), 5.08-5.23 (m, 4H, **H 7, 10**), 5.53-5.59 (m, 2H, **H 15, 20**), 5.65-5.70 (m, 2H, **H 2, 3**), 5.71-5.83 (m, 2H, **H 6, 9**), 6.36 (d, 2H, J = 17.0 Hz, **H 15, 20**), 6.46 (dd, 2H, J = 10.1 Hz, J = 16.6 Hz, **H 14,19**). |

### Application Example 1-2 and Comparative Example 1 (Glass ionomer)

The glass ionomers are composed of a liquid and a glass powder.

### Liquids

The liquids **1-3** were prepared by mixing the individual components as shown in Table 1.

**Table 1: Composition of liquids 1-3**

| **Liquid** | | **1** | **2** | **3** |
|---|---|---|---|---|
| Modified Polycarboxylic acid according to WO 2012/084206, Example 1 | wt-% | 35.000 | 35.000 | 35.000 |
| Hydroxyethyl methacrylate | wt-% | 15.000 | 15.000 | 15.000 |
| N,N'-Diethy)-1,3-propy)ene bisacrylamide | wt-% | 0.000 | 0.000 | 15.000 |
| N,N'-Di(allyl acrylamido)-2-hydroxypropane of Example 1 | wt-% | 15.000 | 0.000 | 0.000 |
| N,N'-(2E)-but-2-en-1,4-diallylbis-[(N-prop-2-en-1) amide of Example 5 | wt-% | 0.000 | 15.000 | 0.000 |
| Water | wt-% | 33.984 | 33.984 | 33.984 |
| Camphor quinone | wt-% | 0.463 | 0.463 | 0.463 |
| Dimethylamino benzonitrile | wt-% | 0.553 | 0.553 | 0.553 |
| Sum | wt-% | 100.000 | 100.000 | 100.000 |

### Glass powder

As glass powder a Strontium aluminum silicate glass with an average particle size of 1.7 µm was used.

### Glass Ionomer

The liquids were mixed 20 to 30 sec with the glass powder in a ratio of 1 to 2.8 (Table 2). Afterwards six rectangular block specimens with the dimensions 2mm x 2mm x 25mm were prepared for every composition by introducing the mixed material into metal molds. These were covered with Melinex foil and pressed between two glass plates. The overall preparation time does not exceed 60 sec. The specimens were cured with a LicuLite Oven for 20 sec from every side. After light curing, the samples were removed from the mold and the edges deflashed with sand paper. They were stored for 1h in a 100% humidity environment at 37°C and afterwards immerged in water at 37°C for 24h. The flexural strength of glass ionomers **1-3** (Table 2) was measured using a Zwick testing machine. The arithmetic average and the standard deviation were calculated from six samples of every composition.

**Table 2: Composition of liquids Glass ionomers 1-3**

| | | **Application Example 1** | **Application Example 2** | **Comparative Example 1** |
|---|---|---|---|---|
| **Glass ionomer** | | **1** | **2** | **3** |
| Liquid of Table 1 | wt-% | 26.316 | 26.316 | 26.316 |
| Glass powder | wt-% | 73.684 | 73.684 | 73.684 |
| Sum | wt-% | 100.000 | 100.000 | 100.000 |
| Flexural strength | MPa | 69.7 ± 3.5 | 65.0 ± 5.7 | 64.7 ± 5.4 |

### Application Example 3 and Comparative Example 2 (Adhesive)

The adhesive mixtures are composed of monomers, an initiator system and a binary solvent system.

### Formulations

The adhesive formulations 1 and 2 were prepared by mixing the individual components as shown in Table 3.

**Table 3: Composition of the adhesive formulations**

| **Formulation #** | | **Application Example 3** | **Comparative Example 2** |
|---|---|---|---|
| **Adhesive** | | **1** | **2** |
| Polyacrylic acid, M_{w}=60 kDa | wt-% | 1.414 | 1.428 |
| Hydroxyethyl methacrylate | wt-% | 4.941 | 4.743 |
| N,N'-Diethyl-1,3-propylene bisacrylamide | wt-% | 0.000 | 45.727 |
| N,N-Di(allyl acrylamido)-2-hydroxypropane of Example 1 | wt-% | 45.902 | 0.000 |
| Ethyl-2-[5-dihydrogenphosporyl-5,2-dioxapentyl] acrylate according to WO 2004/078100, Example 1 | wt-% | 2.331 | 2.368 |
| 2-Propanol | wt-% | 15.079 | 14.806 |
| Water | wt-% | 28.137 | 28.053 |
| Camphor quinone | wt-% | 1.170 | 2.029 |
| Dimethylamino benzonitrile | wt-% | 1.026 | 0.846 |
| Sum | wt-% | 100.000 | 100.000 |

### Preparation

The liquids were mixed in the ratio given in Table 3 and stirred for 2 hours at 22°C in closed vessels and under yellow-light conditions.

### Application

All solutions were applied on pre-conditioned, roughened surfaces (enamel and dentin) of human molar teeth using the following steps: spreading using a applicator microbrush, gently agitating for 20 sec, thoroughly evaporating the solvent for at least 5 sec and finally, blue light curing for 10 sec (minimum output level of 800 mW/cm2). A Spectrum TPH3 (A2) composite post was positioned onto the respective surfaces and blue light cured according to its instructions for use. The specimens were stored in water at 37°C for 24h and subsequently, shear-bond-strengths (Table 4) were determined using a Zwick testing machine. The arithmetic average and the standard deviation were calculated from six samples of every composition and on each template (enamel/dentin), respectively.

| | | **Application Example 3** | **Comparative Example 2** |
|---|---|---|---|
| **Adhesive** | | **1** | **2** |
| **Shear-Bond-Strength on Template** | | | |
| Enamel | MPa | 14.0 ± 2.1 | 6.0 ± 0.6 |
| Dentin | MPa | 13.2 ± 2.3 | 10.2 ± 1.1 |

The adhesion of Comparative Example 2 is only 43 % on enamel and 77 % on dentin compared to Application Example 3.

### Application Example 4 and Comparative Example 3 (Adhesive)

The adhesive mixtures are composed of monomers, an initiator system and a binary solvent system.

### Formulations

The adhesive formulations **1** and **2** were prepared by mixing the individual components as shown in Table 4.

**Table 4: Composition of the adhesive formulations**

| **Formulation #** | | **Application Example 4** | **Comparative Example 3** |
|---|---|---|---|
| **Adhesive** | | **1** | **2** |
| Polyacrylic acid, M_{w}=60 kDa | wt-% | 2.160 | 2.149 |
| Hydroxyethyl methacrylate | wt-% | 6.215 | 6.249 |
| N,N'-Diethyl-1,3-propylene bisacrylamide | wt-% | 0.000 | 44.998 |
| N,N'-(2E)-but-2-en-1,4-diallylbis-[(N-prop-2-en-1) amide of Example 5 | wt-% | 44.807 | 0.000 |
| Ethyl-2-[5-dihydrogenphosporyl-5,2-dioxapentyl] acrylate according to WO 2004/078100, Example 1 | wt-% | 4.139 | 4.142 |
| 10-Methacryloyloxydecyldihydrogenphosphat (MDP, from PCM; Krefeld, Germany) | wt-% | 7.849 | 7.907 |
| 2-Propanol | wt-% | 8.823 | 8.854 |
| Water | wt-% | 23.607 | 23.688 |
| Camphor quinone | wt-% | 1.051 | 1.055 |
| Dimethylamino benzonitrile | wt-% | 0.941 | 0.957 |
| Sum | wt-% | 100.000 | 100.000 |

### Preparation

The liquids were mixed in the ratio given in Table 4 and stirred for 2 hours at 22°C in closed vessels and under yellow-light conditions.

### Application

All solutions were applied on pre-conditioned, roughened surfaces (enamel and dentin) of human molar teeth using the following steps: spreading using a applicator microbrush, gently agitating for 20 sec, thoroughly evaporating the solvent for at least 5 sec and finally, blue light curing for 10 sec (minimum output level of 800 mW/cm2). A Spectrum TPH3 (A2) composite post was positioned onto the respective surfaces and blue light cured according to its instructions for use. The specimens were stored in water at 37°C for 24h and subsequently, shear-bond-strengths (Table 5) were determined using a Zwick testing machine. The arithmetic average and the standard deviation were calculated from six samples of every composition and on each template (enamel/dentin), respectively.

| | | **Application Example 4** | **Comparative Example 3** |
|---|---|---|---|
| **Adhesive** | | **1** | **2** |
| **Shear-Bond-Strength on Template** | | | |
| Enamel | MPa | 17.8 ± 3.9 | 9.6 ± 1.4 |
| Dentin | MPa | 14.4 ± 3.3 | 11.3 ± 1.6 |

The adhesion of Comparative Example 3 is only 54 % on enamel and 78 % on dentin compared to Application Example 4.

## Claims

1. Dental composition comprising a polymerizable compound of the following formula (I):
A-L(B)ₙ (I)
wherein
A is a group of the following formula (II) 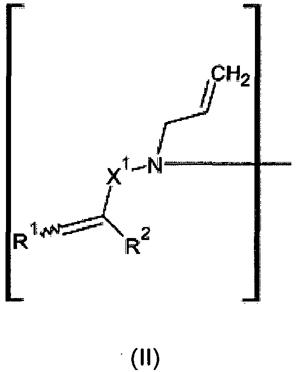
X¹ is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10;
R¹ is a hydrogen atom,
-COOM,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M,
a C₆₋₁₄ aryl or C₃₋₁₄ heteroaryl group which may be substituted by - COOM, -PO₃M, -O-PO₃M₂ or -SO₃M,
R² is a hydrogen atom,
-COOM
a straight chain or branched C₁₋₁₆ alkyl group which may be substituted by a C₆₋₁₄ aryl or C₃₋₁₄ heteroaryl group, -COOM, -PO₃M, -O-PO₃M₂ and -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, or
a C₆₋₁₄ aryl or C₃₋₁₄ heteroaryl group which may be substituted by - COOM, -PO₃M, -O-PO₃M₂ and -SO₃M,
L is a single bond or an (n+1) -valent linker group;
B independently is
a group according to the definition of A,
a group of the following formula (III) 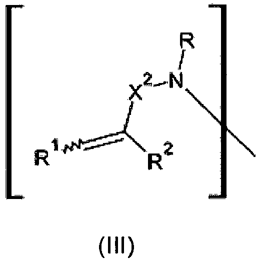 wherein
X² independently has the same meaning as defined for X¹ in formula (II),
R¹ and R² are independent from each other and independently have the same meaning as defined for formula (II),
R is a hydrogen atom,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, - PO₃M, -O-PO₃M₂ or -SO₃M,
a C₆₋₁₄ aryl or C₃₋₁₄ heteroaryl group which may be substituted by -COOM, -PO₃M, -O-PO₃M₂ or -SO₃M,
a group of the following formula (IV) 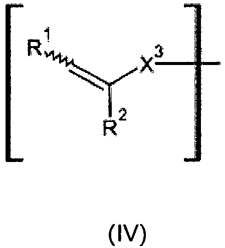 wherein
X³ is CO, -CH₂CO-, CS, or -CH₂CS-,
R¹ and R² which are independent from each other and independently have the same meaning as defined for formula (II), or
a group [Z'X"]ₘE,
wherein
Z' is a straight chain or branched C₁₋₄ alkylene group,
X" is an oxygen atom, a sulfur atom or NH,
E is a hydrogen atom,
PO₃M₂,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, - PO₃M, -O-PO₃M₂ or -SO₃M,
a C₆₋₁₄ aryl or C₃₋₁₄ heteroaryl group which may be substituted by -COOM, -PO₃M, -O-PO₃M₂ or -SO₃M, and
m is an integer of from 1 to 10; and
n is an integer of from from 1 to 4;
wherein M which are independent from each other each represent a hydrogen atom or a metal atom;
provided that in case L is a single bond, B cannot be a group according to the definition of A or a group of the formula (III), and provided that the compound of formula (I) is not N,N-diallylacrylamide.

2. The dental composition according to claim 1, wherein n is 1.

3. The dental composition according to claim 1 or 2, wherein L is a single bond.

4. The dental composition according to claim 1 or 2, wherein L is a linker group.

5. The dental composition according to any one of the preceding claims wherein X¹ is CO.

6. The dental composition according to any one of claims 1, 2, 4 or 5, wherein the linker group of L is a C₁₋₁₂ hydrocarbon group which may contain 1 to 3 carbonyl groups or heteroatoms selected from oxygen, nitrogen and sulfur, and which may be substituted by a hydroxyl group, a C₆₋₁₄ aryl group, -COOM, -PO₃M, -O-PO₃M₂ or -SO₃M, wherein M is a hydrogen atom or a metal atom.

7. The dental composition according to claim 6, wherein the C₁₋₁₂ hydrocarbon group is a group of the following formula (V), wherein
-Y-L'-Y'- (V)
wherein Y and Y' independently represent a single bond or CO or CS and L' is a C₁₋₁₂ alkylene group, a C₃₋₁₂ cycloalkylene group or a C₁₋₁₂ alkenylene group.

8. The dental composition according to claim 7, wherein X¹ is CO.

9. The dental composition according to claim 7, wherein Y is CO.

10. The dental composition according to claim 7, wherein R¹ is a hydrogen atom and R² is a hydrogen atom or a methyl group.

11. The dental composition according to claim 4, wherein the linker group is a polyamide residue of the following formula (V)
-Y-L'-Y'- (V)
wherein
Y and Y' represent CO and the polyamide moiety of formula (V) is obtainable by a process comprising the step of a step-growth polymerization of a mixture containing a diamine of the following formula (VI) and a compound of the following formula (VII) having at least two carboxylic acid groups,
R³(NHR")_{y} (VI)
wherein
R³ represents an y-valent C₂₋₂₀ straight-chain, branched or cyclic hydrocarbon group which may optionally contain from 1 to 6 hetero atoms selected from nitrogen, oxygen, or sulphur atoms in the backbone of the hydrocarbon group, and optionally from 1 to 6 functional groups selected from hydroxyl groups, thiol groups and amino groups;
R" represents a hydrogen atom, an allyl group a straight chain or branched C₁₋₆ alkyl group which may be substituted by a C₃₋₆ cycloalkyl group, or a C₆₋₁₄ aryl group, a C₃₋₆ cycloalkyl group which may be substituted by a C₁₋₆ alkyl group or a C₆₋₁₄ aryl group; and
y represents an integer of at least 2;
MOOC-R⁴-COOM (VII)
wherein R⁴ represents a C₁₋₂₀ straight-chain, branched, cyclic or aromatic hydrocarbon group which may optionally contain a carbon-carbon double bond, from 1 to 6 heteroatoms selected from nitrogen, oxygen, or sulphur atoms in the backbone of the hydrocarbon group, and optionally from 1 to 6 functional groups selected from carboxylic acid groups, hydroxyl groups, thiol groups and amino groups, and the M which may be the same or different, independently represent a hydrogen atom or a metal atom.

12. The dental composition according to any one of the preceding claims, wherein at least one of X¹ and Y is CO.

13. The dental composition according to any one of the preceding claims, wherein the compound of formula (I) has an average molecular weight of from 130 to 10,000, and/or which is selected from a dental adhesive composition, a bonding agent, a pit and fissure sealant, a dental desensitizing composition, a pulp capping composition, a dental composite, dental glass ionomer cement, a dental cement, a dental root canal sealer composition, and a dental infiltrant.

14. The dental composition according to any one of the claims 1 or 3, wherein B is not a group of formula (II) or (III).

15. Use of a compound of formula (I) as defined in claim 1, for the preparation of a dental composition selected from a dental adhesive composition, a bonding agent, a pit and fissure sealant, a dental desensitizing composition, a pulp capping composition, a dental composite, dental glass ionomer cement, a dental cement, a dental root canal sealer composition, and a dental infiltrant.

## Patentansprüche

1. Dentale Zusammensetzung, umfassend eine polymerisierbare Verbindung der folgenden Formel (I):
A-L(B)ₙ (I),
wobei
A für eine Gruppe der folgenden Formel (II) steht 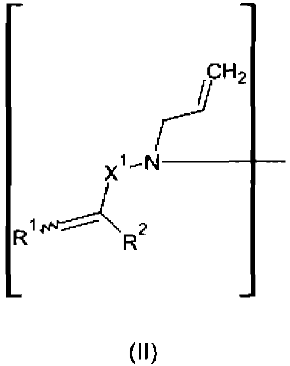
X¹ für CO, CS, CH₂ oder eine Gruppe [X'Z]ₖ steht, wobei X' für ein Sauerstoffatom, ein Schwefelatom oder NH steht, Z für eine geradkettige oder verzweigte C₁₋₄ Alkylengruppe steht und k für eine ganze Zahl von 1 bis 10 steht;
R¹ für ein Wasserstoffatom steht,
-COOM,
eine geradkettige oder verzweigte C₁₋₁₆ Alkylgruppe, die mit einer C₃₋₆ Cycloalkylgruppe, einer C₆₋₁₄ Aryl- oder C₃₋₁₄ Heteroarylgruppe, -COOM, - PO₃M, -O-PO₃M₂ oder -SO₃M substituiert sein kann,
eine C₃₋₆ Cycloalkylgruppe, die mit einer C₁₋₁₆ Alkylgruppe, einer C₆₋₁₄ Aryl- oder C₃₋₁₄ Heteroarylgruppe, -COOM, -PO₃M, -O-PO₃M₂ oder -SO₃M substituiert sein kann,
eine C₆₋₁₄ Aryl- oder C₃₋₁₄ Heteroarylgruppe, die mit -COOM, -PO₃M, -O-PO₃M₂ oder -SO₃M substituiert sein kann,
R² für ein Wasserstoffatom steht,
-COOM,
eine geradkettige oder verzweigte C₁₋₁₆ Alkylgruppe, die mit einer C₆₋₁₄ Aryl- oder C₃₋₁₄ Heteroarylgruppe, -COOM, -PO₃M, -O-PO₃M₂ und -SO₃M substituiert sein kann, eine C₃₋₆ Cycloalkylgruppe, die mit einer C₁₋₁₆ Alkylgruppe, einer C₆₋₁₄ Aryl- oder C₃₋₁₄ Heteroarylgruppe, -COOM, -PO₃M, -O-PO₃M₂ oder -SO₃M substituiert sein kann, oder
eine C₆₋₁₄ Aryl- oder C₃₋₁₄ Heteroarylgruppe, die mit -COOM, -PO₃M, -O-PO₃M₂ und -SO₃M substituiert sein kann,
L für eine Einfachbindung oder eine (n+1)-wertige Linkergruppe steht;
B unabhängig für
eine Gruppe gemäß der Definition von A steht,
eine Gruppe der folgenden Formel (III) 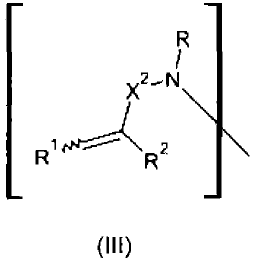 wobei
X² unabhängig die gleiche Bedeutung wie für X¹ in Formel (II) definiert aufweist,
R¹ und R² unabhängig voneinander sind und unabhängig die gleiche Bedeutung wie für Formel (II) definiert aufweisen,
R für ein Wasserstoffatom steht,
eine geradkettige oder verzweigte C₁₋₁₆ Alkylgruppe, die mit einer C3-6 Cycloalkylgruppe, einer C₆₋₁₄ Aryl- oder C₃₋₁₄ Heteroarylgruppe, - COOM, -PO₃M, -O-PO₃M₂ oder -SO₃M substituiert sein kann,
eine C₃₋₆ Cycloalkylgruppe, die mit einer C₁₋₁₆ Alkylgruppe, einer C₆₋₁₄ Aryl- oder C₃₋₁₄ Heteroarylgruppe, -COOM, -PO₃M, -O-PO₃M₂ oder - SO₃M substituiert sein kann,
eine C₆₋₁₄ Aryl- oder C₃₋₁₄ Heteroarylgruppe, die mit -COOM, -PO₃M, - O-PO₃M₂ oder -SO₃M substituiert sein kann,
eine Gruppe der folgenden Formel (IV) 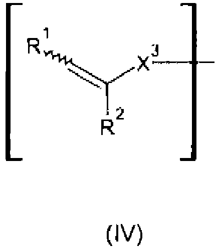 wobei
X³ für CO, -CH₂CO-, CS oder -CH₂CS- steht, R¹ und R², die unabhängig voneinander sind und unabhängig die gleiche Bedeutung wie für Formel (II)definiert aufweisen oder
eine Gruppe [Z'X"]ₘE,
wobei
Z' für eine geradkettige oder verzweigte C₁₋₄ Alkylengruppe steht,
X" für ein Sauerstoffatom, ein Schwefelatom oder NH steht,
E für ein Wasserstoffatom steht,
PO₃M₂,
eine geradkettige oder verzweigte C₁₋₁₆ Alkylgruppe, die mit einer C₃₋₆ Cycloalkylgruppe, einer C₆₋₁₄ Aryl- oder C₃₋₁₄ Heteroarylgruppe, - COOM, -PO₃M, -O-PO₃M₂ oder -SO₃M substituiert sein kann,
eine C₃₋₆ Cycloalkylgruppe, die mit einer C₁₋₁₆ Alkylgruppe, einer C₆₋₁₄ Aryl- oder C₃₋₁₄ Heteroarylgruppe, -COOM, -PO₃M, -O-PO₃M₂ oder - SO₃M substituiert sein kann,
eine C₆₋₁₄ Aryl- oder C₃₋₁₄ Heteroarylgruppe, die mit -COOM, -PO₃M, -
O-PO₃M₂ oder -SO₃M substituiert sein kann, und
m für eine ganze Zahl von 1 bis 10 steht; und
n für eine ganze Zahl von 1 bis 4 steht;
wobei M, welche voneinander unabhängig sind, jeweils für ein Wasserstoffatom oder ein Metallatom stehen;
mit der Maßgabe, dass in dem Fall, in dem L für eine Einfachbindung steht, B keine Gruppe gemäß der Definition von A oder eine Gruppe der Formel (III) sein kann und mit der Maßgabe, dass die Verbindung der Formel (I) nicht N,N-Diallylacrylamid ist.

2. Dentale Zusammensetzung nach Anspruch 1, wobei n 1 ist.

3. Dentale Zusammensetzung nach Anspruch 1 oder 2, wobei L eine Einfachbindung ist.

4. Dentale Zusammensetzung nach nach Anspruch 1 oder 2, wobei L eine Linkergruppe ist.

5. Dentale Zusammensetzung nach einem der vorhergehenden Ansprüche, wobei X¹ für CO steht.

6. Dentale Zusammensetzung nach einem der Ansprüche 1, 2, 4 oder 5, wobei die Linkergruppe von L für eine C₁₋₁₂ Kohlenwasserstoffgruppe steht, die 1 bis 3 Carbonylgruppen oder Heteroatome enthalten kann, ausgewählt aus Sauerstoff, Stickstoff und Schwefel, und die mit einer Hydroxylgruppe, einer C₆₋₁₄ Arylgruppe, -COOM, -PO₃M,-O-PO₃M₂ oder -SO₃M substituiert sein kann, wobei M für ein Wasserstoffatom oder ein Metallatom steht.

7. Dentale Zusammensetzung nach Anspruch 6, wobei die C₁₋₁₂ Kohlenwasserstoffgruppe für eine Gruppe der folgenden Formel (V) steht
-Y-L'-Y'- (V).
wobei Y und Y' unabhängig für eine Einfachbindung oder CO oder CS stehen und L' für eine C₁₋₁₂ Alkylengruppe, eine C₃₋₁₂ Cycloalkylengruppe oder eine C₁₋₁₂ Alkenylengruppe steht.

8. Dentale Zusammensetzung nach Anspruch 7, wobei X¹ für CO steht.

9. Dentale Zusammensetzung nach Anspruch 7, wobei Y für CO steht.

10. Dentale Zusammensetzung nach Anspruch 7, wobei R¹ für ein Wasserstoffatom steht und R² für ein Wasserstoffatom oder eine Methylgruppe steht.

11. Dentale Zusammensetzung nach Anspruch 4, wobei die Linkergruppe ein Polyamidrest der folgenden Formel (V) ist:
-Y-L'-Y'- (V),
wobei Y und Y' für CO stehen und der Polyamidrest der Formel (V) durch ein Verfahren erhältlich ist, das einen Schritt einer Stufenwachstumspolymerisation einer Mischung umfasst, die ein Diamin der folgenden Formel (VI) und eine Verbindung der folgenden Formel (VII), die wenigstens zwei Carbonsäuregruppen aufweist, enthält,
R³(NHR")_{y} (VI),
wobei
R³ für eine y-wertige C₂₋₂₀ geradkettige, verzweigte oder cyclische Kohlenwasserstoffgruppe steht, die wahlweise von 1 bis 6 Heteroatome enthalten kann, ausgewählt aus Stickstoff-, Sauerstoff- oder Schwefelatomen in dem Gerüst der Kohlenwasserstoffgruppe, und wahlweise von 1 bis 6 funktionelle Gruppen, ausgewählt aus Hydroxylgruppen, Thiolgruppen und Aminogruppen;
R" für ein Wasserstoffatom steht, eine Allylgruppe, eine geradkettige oder verzweigte C₁₋₆ Alkylgruppe, die mit einer C₃₋₆ Cycloalkylgruppe oder einer C₆₋₁₄ Arylgruppe substituiert sein kann, eine C₃₋₆ Cycloalkylgruppe, die mit einer C₁₋₆ Alkylgruppe oder einer C₆₋₁₄ Arylgruppe substituiert sein kann; und
y für eine ganze Zahl von wenigstens 2 steht;
MOOC-R⁴-COOM (VII)
wobei R⁴ für eine C₁₋₂₀ geradkettige, verzweigte, cyclische oder aromatische Kohlenwasserstoffgruppe steht, die wahlweise eine Kohlenstoff-Kohlenstoff-Doppelbindung enthalten kann, von 1 bis 6 Heteroatome, ausgewählt aus Stickstoff-, Sauerstoff- oder Schwefelatomen in dem Gerüst der Kohlenwasserstoffgruppe, und wahlweise von 1 bis 6 funktionelle Gruppen, ausgewählt aus Carbonsäuregruppen, Hydroxylgruppen, Thiolgruppen und Aminogruppen, und M, das gleich oder verschieden sein kann, unabhängig für ein Wasserstoffatom oder ein Metallatom steht.

12. Dentale Zusammensetzung nach einem der vorhergehenden Ansprüche, wobei wenigstens eines von X¹ und Y für CO steht.

13. Dentale Zusammensetzung nach einem der vorhergehenden Ansprüche, wobei die Verbindung der Formel (1) ein durchschnittliches Molekulargewicht von 130 bis 10.000 aufweist und/oder welche ausgewählt ist aus einer Zusammensetzung eines Dentinadhäsivs, einem Haftvermittler, einem Grübchen- und Fissurenversiegler, einer dentalen Zusammensetzung zur Desensibilisierung, einer Zusammensetzung für die Pulpa-Überkappung, einem dentalen Komposit, einem dentalen Glasionomer-Zement, einem Zahnzement, einer Zusammensetzung für die Versiegelung von Zahnwurzelkanälen und einem dentalen Mittel zur Infiltration.

14. Dentale Zusammensetzung nach einem der Ansprüche 1 oder 3, wobei B keine Gruppe der Formel (II) oder (III) ist.

15. Verwendung einer wie in Anspruch 1 definierten Verbindung der Formel (I) zur Herstellung einer dentalen Zusammensetzung, ausgewählt aus einer Zusammensetzung eines Dentinadhäsivs, einem Haftvermittler, einem Grübchen- und Fissurenversiegler, einer dentalen Zusammensetzung zur Desensibilisierung, einer Zusammensetzung für die Pulpa-Überkappung, einem dentalen Komposit, einem dentalen Glasionomer-Zement, einem Zahnzement, einer Zusammensetzung für die Versiegelung von Zahnwurzelkanälen und einem dentalen Mittel zur Infiltration.

## Revendications

1. Composition dentaire comprenant un composé polymérisable de formule (I) suivante :
A-L(B)ₙ (I)
dans laquelle
A est un groupe de formule (II) suivante : 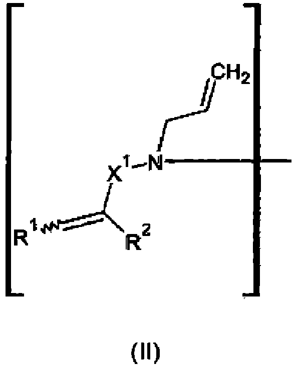
X¹ est CO, CS, CH₂ ou un groupe [X'Z]ₖ où X' est un atome d'oxygène, un atome de soufre ou NH, Z est un groupe alkylène en C₁ à C₄ linéaire ou ramifié, et k est un entier de 1 à 10 ; R¹ est un atome d'hydrogène,
-COOM,
un groupe alkyle en C₁ à C₁₆ linéaire ou ramifié qui peut être substitué par un groupe cycloalkyle en C₃ à C₆, un groupe aryle en C₆ à C₁₄ ou hétéroaryle en C₃ à C₁₄, -COOM, -PO₃M, -O-PO₃M₂ ou -SO₃M,
un groupe cycloalkyle en C₃ à C₆ qui peut être substitué par un groupe alkyle en C₁ à C₁₆, un groupe aryle en C₆ à C₁₄ ou hétéroaryle en C₃ à C₁₄, -COOM, -PO₃M, -O-PO₃M₂ ou -SO₃M, un groupe aryle en C₆ à C₁₄ ou hétéroaryle en C₃ à C₁₄ qui peut être substitué par -COOM, -PO₃M, -O-PO₃M₂ ou -SO₃M,
R² est un atome d'hydrogène,
-COOM,
un groupe alkyle en C₁ à C₁₆ linéaire ou ramifié qui peut être substitué par un groupe aryle en C₆ à C₁₄ ou hétéroaryle en C₃ à C₁₄, -COOM, -PO₃M, -O-PO₃M₂ ou -SO₃M, un groupe cycloalkyle en C₃ à C₆ qui peut être substitué par un groupe alkyle en C₁ à C₁₆, un groupe aryle en C₆ à C₁₄ ou hétéroaryle en C₃ à C₁₄, -COOM, -PO₃M, -O-PO₃M₂ ou -SO₃M, ou un groupe aryle en C₆ à C₁₄ ou hétéroaryle en C₃ à C₁₄ qui peut être substitué par -COOM, -PO₃M, -O-PO₃M₂ ou -SO₃M,
L est une liaison simple ou un groupe lieur de valence (n+1) ; B est indépendamment
un groupe conforme à la définition de A,
un groupe de formule (III) suivante : 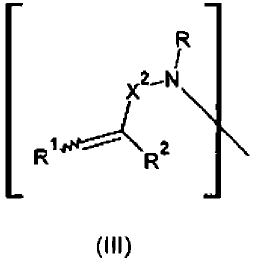 dans laquelle
X² a indépendamment la même signification que celle définie pour X¹ dans la formule (II),
R¹ et R² sont indépendants l'un de l'autre et ont indépendamment la même signification que celle définie à propos de la formule (II),
R est un atome d'hydrogène,
un groupe alkyle en C₁ à C₁₆ linéaire ou ramifié qui peut être substitué par un groupe cycloalkyle en C₃ à C₆, un groupe aryle en C₆ à C₁₄ ou hétéroaryle en C₃ à C₁₄, -COOM, -PO₃M, -O-PO₃M₂ ou -SO₃M,
un groupe cycloalkyle en C₃ à C₆ qui peut être substitué par un groupe alkyle en C₁ à C₁₆, un groupe aryle en C₆ à C₁₄ ou hétéroaryle en C₃ à C₁₄, -COOM, -PO₃M, -O-PO₃M₂ ou -SO₃M, un groupe aryle en C₆ à C₁₄ ou hétéroaryle en C₃ à C₁₄ qui peut être substitué par -COOM, -PO₃M, -O-PO₃M₂ ou -SO₃M,
un groupe de formule (IV) suivante : 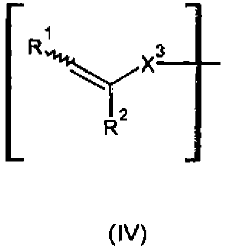
dans laquelle X³ est CO, -CH₂CO-, CS, ou -CH₂CS-,
R¹ et R² sont indépendants l'un de l'autre et ont indépendamment la même signification que celle définie à propos de la formule (II), ou
un groupe [Z'X"]ₘE, dans lequel
Z' est un groupe alkylène en C₁ à C₄ linéaire ou ramifié, X" est un atome d'oxygène, un atome de soufre ou NH, E est un atome d'hydrogène,
PO₃M₂ , un groupe alkyle en C₁ à C₁₆ linéaire ou ramifié qui peut être substitué par un groupe cycloalkyle en C₃ à C₆, un groupe aryle en C₆ à C₁₄ ou hétéroaryle en C₃ à C₁₄, -COOM,-PO₃M, -O-PO₃M₂ ou -SO₃M,
un groupe cycloalkyle en C₃ à C₆ qui peut être substitué par un groupe alkyle en C₁ à C₁₆, un groupe aryle en C₆ à C₁₄ ou hétéroaryle en C₃ à C₁₄, -COOM, -PO₃M, -O-PO₃M₂ ou -SO₃M, un groupe aryle en C₆ à C₁₄ ou hétéroaryle en C₃ à C₁₄ qui peut être substitué par -COOM, -PO₃M, -O-PO₃M₂ ou -SO₃M, et
m est un entier de 1 à 10 ; et
n est un entier de 1 à 4 ;
où chacun des M, qui sont indépendants les uns des autres, représente un atome d'hydrogène ou un atome de métal ; sous réserve que, dans le cas où L est une liaison simple, B ne puisse pas être un groupe conforme à la définition A ou un groupe de formule (III), et sous réserve que le composé de formule (I) ne soit pas le N,N-diallylacrylamide.

2. Composition dentaire selon la revendication 1, dans laquelle n vaut 1.

3. Composition dentaire selon la revendication 1 ou 2, dans laquelle L est une liaison simple.

4. Composition dentaire selon la revendication 1 ou 2, dans laquelle L est un groupe lieur.

5. Composition dentaire selon l'une quelconque des revendications précédentes, dans laquelle X¹ est CO.

6. Composition dentaire selon l'une quelconque des revendications 1, 2, 4 ou 5, dans laquelle le groupe lieur de L est un groupe hydrocarboné en C₁ à C₁₂ qui peut contenir 1 à 3 groupes carbonyle ou hétéroatomes choisis parmi l'oxygène, l'azote et le soufre, et qui peut être substitué par un groupe hydroxyle, un groupe aryle en C₆ à C₁₄, -COOM, -PO₃M, -O-PO₃M₂ ou -SO₃M, où M est un atome d'hydrogène ou un atome de métal.

7. Composition dentaire selon la revendication 6, dans laquelle le groupe hydrocarboné en C₁ à C₁₂ est un groupe de formule (V) suivante :
-Y-L'-Y' (V)
dans laquelle Y et Y¹ représentent indépendamment une liaison simple ou CO ou CS et L' est un groupe alkylène en C₁ à C₁₂, un groupe cycloalkylène en C₃ à C₁₂ ou un groupe alcénylène en C₁ à C₁₂.

8. Composition dentaire selon la revendication 7, dans laquelle X¹ est CO.

9. Composition dentaire selon la revendication 7, dans laquelle Y est CO.

10. Composition dentaire selon la revendication 7, dans laquelle R¹ est un atome d'hydrogène et R² est un atome d'hydrogène ou un groupe méthyle.

11. Composition dentaire selon la revendication 4, dans laquelle le groupe lieur est un résidu de polyamide de formule (V) suivante :
-Y-L'-Y' (V)
dans laquelle
Y et Y' représentent CO et le fragment de polyamide de formule (V) peut être obtenu par un procédé comprenant une étape de polycondensation d'un mélange contenant une diamine de formule (VI) suivante et un composé de formule (VII) suivante ayant au moins deux groupes acide carboxylique,
R³ (NHR")_{y} (VI)
dans laquelle
R³ représente un groupe hydrocarboné linéaire, ramifié ou cyclique en C₂ à C₂₀ de valence y qui peut éventuellement contenir 1 à 6 hétéroatomes choisis parmi les atomes d'azote, d'oxygène et de soufre dans la charpente du groupe hydrocarboné, et éventuellement 1 à 6 groupes fonctionnels choisis parmi les groupes hydroxyle, les groupes thiol et les groupes amino ;
R" représente un atome d'hydrogène, un groupe allyle, un groupe alkyle en C₁ à C₆ linéaire ou ramifié qui peut être substitué par un groupe cycloalkyle en C₃ à C₆ ou un groupe aryle en C₆ à C₁₄, un groupe cycloalkyle en C₃ à C₆ qui peut être substitué par un groupe alkyle en C₁ à C₆ ou un groupe aryle en C₆ à C₁₄ ; et
y est un entier valant au moins 2 ;
MOOC-R⁴-COOM (VII)
dans laquelle R⁴ représente un groupe hydrocarboné linéaire, ramifié, cyclique ou aromatique en C₁ à C₂₀ qui peut éventuellement contenir une double liaison carbone-carbone, 1 à 6 hétéroatomes choisis parmi les atomes d'azote, d'oxygène et de soufre dans la charpente du groupe hydrocarboné, et éventuellement 1 à 6 groupes fonctionnels choisis parmi les groupes acide carboxylique, les groupes hydroxyle, les groupes thiol et les groupes amino, et les M, qui peuvent être identiques ou différents, représentent indépendamment un atome d'hydrogène ou un atome de métal.

12. Composition dentaire selon l'une quelconque des revendications précédentes, dans laquelle au moins l'un parmi X¹ et Y est CO.

13. Composition dentaire selon l'une quelconque des revendications précédentes, dans laquelle le composé de formule (I) a une masse moléculaire moyenne de 130 à 10 000, et/ou qui est choisie parmi une composition dentaire adhésive, un agent collant, une résine pour puits et fissures, une composition dentaire désensibilisante, une composition de coiffage pulpaire, un composite dentaire, un ciment de verre ionomère dentaire, un ciment dentaire, une composition dentaire d'obturateur de canal radiculaire, et une composition dentaire d'infiltration.

14. Composition dentaire selon l'une quelconque des revendications 1 et 3, dans laquelle B n'est pas un groupe de formule (II) ou (III).

15. Utilisation d'un composé de formule (I) tel que défini dans la revendication 1, pour la préparation d'une composition dentaire choisie parmi une composition dentaire adhésive, un agent collant, une résine pour puits et fissures, une composition dentaire désensibilisante, une composition de coiffage pulpaire, un composite dentaire, un ciment de verre ionomère dentaire, un ciment dentaire, une composition dentaire d'obturateur de canal radiculaire, et une composition dentaire d'infiltration.
